# EUROPÄISCHE PATENTSCHRIFT

(11) **EP 0 914 598 B1**
(45) Veröffentlichungstag und Bekanntmachung des Hinweises auf die Patenterteilung: **04.06.2003**
(21) Anmeldenummer: 97920523.4
(22) Anmeldetag: 18.03.1997
(51) Int. Cl.: G01N 1/40, G01N 33/49

(54) **VORRICHTUNG FÜR DAS SAMMELN UND/ODER ABTRENNEN VON PARTIKELN AUS EINER FLÜSSIGKEIT FÜR DIE MEDIZINISCHE DIAGNOSE UND/ODER TECHNISCHE FILTRATION**
DEVICE FOR COLLECTING AND/OR SEPARATING PARTICLES FROM A FLUID FOR MEDICAL DIAGNOSTIC PURPOSES AND/OR TECHNICAL FILTRATION
DISPOSITIF POUR COLLECTER ET/OU SEPARER DES PARTICULES CONTENUES DANS UN LIQUIDE A DES FINS DE DIAGNOSTIC MEDICAL ET/OU DE FILTRATION TECHNIQUE

(30) Priorität: 29.03.1996 DE 19612487
(43) Veröffentlichungstag der Anmeldung: 12.05.1999
(73) Patentinhaber: Schlüter, Gert, 79346 Endingen (DE)
(72) Erfinder: Schlüter, Gert, 79346 Endingen (DE)
(74) Vertreter: Flügge, Christian
(86) Internationale Anmeldenummer: DE9700548
(87) Internationale Veröffentlichungsnummer: WO97037207

(56) Entgegenhaltungen:
- EP-A- 0 120 187
- EP-A- 0 294 645
- EP-A- 0 382 905
- WO-A-92/17110
- WO-A-93/01306

## Beschreibung

Die Erfindung betrifft eine Vorrichtung für das Sammeln und/oder Abtrennen von Partikeln aus einer Flüssigkeit für die medizinische Diagnose und/oder technische Filtration, aufweisend ein von einer beiderends offenen Filterhülse festgelegtes Filterelement.

Zum Sammeln und/oder Abtrennen von Partikeln aus einer Flüssigkeit für z.B. die medizinische Diagnose werden häufig Zentrifugen verwandt, d.h. die Flüssigkeitsproben werden zur Gewinnung der Partikel zentrifugiert.

Das Sediment wird dann über Objektträgerausstriche ausgewertet.

Bei dieser Methode kommt es jedoch häufig zu Veränderungen der zu diagnostizierenden Partikel und darüber hinaus erfordert diese Methode einen hohen technischen Aufwand, d.h. Apparateaufwand und ist daher in der Praxis zum einen zu aufwendig und zum anderen zu teuer.

Um diese Nachteile zu beseitigen wurden Filtrationsverfahren entwickelt, die sich z.B. die kapillare Wirkung einer Saugeinlage zu nutze machen, d.h. es wird ein Filterbehälter mit einer unteren Öffnung in die zu untersuchende Flüssigkeit gestellt, so daß mittels der kapillaren Wirkung die Flüssigkeit in der Saugeinlage aufsteigt und die zu untersuchenden Partikel am unteren Ende der Saugeinlage, d.h. an einem dort angeordneten zellenundurchlässigen Zellträgerelement verbleiben, wobei nach Sättigung der Saugeinlage die Vorrichtung aus der zu untersuchenden Flüssigkeit genommen werden kann.

Die auf dem zellundurchlässigen Zellträgerelement angelagerten Partikel bilden jedoch zum einen keinen repräsentativen Querschnitt der in der Flüssigkeit vorhandenen Partikel, da eine Durchmischung mit der Vorrichtung oder einem Gegenstand nicht möglich ist, insbesondere da die Partikel lediglich durch die, durch die Kapillaren entstandene Saugwirkung an dem Zellträgerelement haften und so permanent die Gefahr eine Abspühlung der Partikel vom Zellträgerelement besteht, insbesondere bei Herausnahme der Vorrichtung aus der zu untersuchenden Flüssigkeit, d.h. das auch hier die Gefahr besteht, das ein Großteil oder auch alle Partikel von dem Zellträgerelement abgeschwemmt werden.

Aus der EP-A-0 120 187 ist eine Vorrichtung bekannt, die der Aufnahme, dem Transport und der Gewinnung von in Körperflüssigkeiten, zellfixierenden Flüssigkeiten und Mischungen von Körperflüssigkeiten und zellfixierenden Flüssigkeiten befindlichen Zellmaterials sowie der Übertragung des gewonnen Zellmaterials auf Objektträger dient. Dort wird ein Faserelement als Filterelement verwendet, das in einer beiderends offenen Filterhülse, einer Hülle festgelegt und gehalten ist. Das Faserelement ist mit seiner Hülle weiter einerends in dem Auslaß eines Gefäßes wandbündig herausnehmbar angeordnet. Andernends ist das Faserelement mit Hülle wandbündig in einer Hülse gefaßt, die auf der Seite, auf der das Faserelement mit Hülle in sie hineinragt, die gleiche Querschnittsfläche und den gleichen Querschnitt wie die des Auslasses aufweist, wobei das Faserelement mit der Hülse flüssigkeits- und gasdicht fest verbunden ist. Das Faserelement steht weiter der Hülle axial beiderends vor. Die Hülse, das Faserelement mit Hülle und das Gefäß sowie Stopfen und Kappen bilden eine funktionelle Einheit.

Die WO-A-93/01306 offenbart ein Verfahren der Granulozytendiagnose, bei dem eine Diagnosevorrichtung Verwendung findet, die ringförmig aufgebaut einerends eine Fassung für eine eine in einem Lösungsmittel verdünnte Blutprobe enthaltene Küvette aufweist. Andernends erfolgt der Anschluß einer Injektionsspritze über einen Adapter, mittels der ein Druckgefälle aufgebaut werden kann. Infolgedessen wird der Inhalt der Küvette über einen trichterartig ausgebildeten Abschnitt einer Durchgangsbohrung der Diagnosevorrichtung zu einer Membran abfließen, an der sich die Flüssigkeit und in der Flüssigkeit enthaltene Partikel scheiden. Die Flüssigkeit durchsetzt die Membran und wird in einem Absorber aus einem flüssigkeitsspeichernden Material aufgenommen, der in einer Zylinderbohrung größeren Durchmessers als der der Membran angeordnet ist. Diese Zylinderbohrung weitet sich nach Außen der Diagnosevorrichtung für die Aufnahme des Adapters für die Injektionsspritze dann nochmals auf.

Aus EP-A-0 382 905 ist eine Vorrichtung für das Sammeln und Abtrennen von Partikeln aus einer Flüssigkeit für die medizienische Diagnose und/oder technische Filtration bekannt. Diese weist ein von einer beiderends offenen Filterhülse festgelegtes Filterelement auf.

Der Erfindung liegt daher die Aufgabe zugrunde, eine Vorrichtung zur Verfügung zu stellen, die dem Anwender bzw. Benutzer die Möglichkeit zum Sammeln und/oder Abtrennen von Partikeln aus einer Flüssigkeit für die medizinische Diagnose und/oder technische Filtration an die Hand gibt, die sich durch eine einfache Handhabung, hohe Effizienz und kostengünstige Benutzung sowie Herstellungsmöglichkeit auszeichnet, insbesondere eine fraktionierte Abtrennung kleinster und größerer Zellkörper aus Flüssigkeiten quantitativ und qualitativ einwandfrei ermöglicht und darüber hinaus die Möglichkeit bietet, die gewonnenen Zellkörper oder Partikel auf Flächen, wie z.B. Objektträger zu deponieren, ohne daß es eines weiteren Zwischenschrittes bedarf, womit eine Vielzahl von Einsatzmöglichkeiten bei einem einfachen konstruktiven Aufbau der Vorrichtung ermöglicht ist.

Diese Aufgabe wird durch eine Vorrichtung der eingangs genannten Gattung mit den Merkmalen des unabhängigen Patentanspruchs 1 gelöst. Weitere vorteilhafte Ausgestaltungen der Vorrichtung sind Gegenstand der Unteransprüche 2 bis 19.

Dadurch, daß eine Vorrichtung bereitgestellt wird, bei der ein Filterelement vorgesehen ist und das Filterelement in einer eine Kraftbeaufschlagung auf das Filterelement ermöglichenden Filterhülse angeordnet ist, wird die Möglichkeit geschaffen, Partikel aus einer Flüssigkeit für die medizinische Diagnose und/oder technische Filtration zu sammeln und/oder abzutrennen, ohne daß es eines großen Apparateaufbaues bedarf, z.B. des Einsatzes einer Zentrifuge.

Weist die Filterhülse einen das Filterelement wenigstens teilweise in direktem Kontakt umschließenden Abschnitt auf und ist daran anschließend ein, dem der Filterfront abgewandten Ende des Filterelements zugeordneter, ein das Filterelement wenigstens teilweise im Abstand umhüllender Abschnitt vorgesehen, ist sichergestellt, daß das Filterelement auf einfache, aber um so praktikabeler Weise in der Filterhülse festgelegt ist.

Da der anschließende Abschnitt der Filterhülse wenigstens eine, bevorzugt zwei umlaufende Dichtlippe aufweist, so wird dadurch zum einen bspw. die einfache Plazierung eines Hohlstopfens gewährleistet und zum anderen im Zusammenspiel mit dem Hohlstopfen sichergestellt, daß vor der Filterfront ein flüssigkeitsdicht abgeschlossener Raum entsteht, der eine Ablagerung der Partikel auf der Filterfront ermöglichend ausgestaltet ist.

Auch ein flüssigkeitsdichtes Ankoppeln eines Reagenzröhrchens oder dergleichen ist so ermöglicht.

Insbesondere kann jedoch der das Filterelement wenigstens teilweise in direktem Kontakt umschließende Abschnitt einer ersten Vorrichtung mit dem die Filterhülse wenigstens teilweise im Abstand umhüllenden Abschnitt einer zweiten Vorrichtung gekoppelt werden. Es wird dadurch erreicht, daß z.B. zwei oder auch mehr Vorrichtungen in Reihe geschaltet werden können, die z.B. unterschiedliche Filterfrontöffnungen aufweisen, so daß Partikel verschiedener Größe in einem Arbeitsgang auf die, in Reihe geschalteten Filterfronten abgelagert werden können bzw. von diesen ausgesondert werden können.

Weist die Vorrichtung bevorzugt im Bereich der Filterfront eine oder zwei die Kapillarität des Filterelements partiell, wenigstens im Bereich der Filterfront verändernde Einrichtungen auf, so ist es dadurch möglich, die Strömungsverhältnisse innerhalb des Filterelmentes in optimaler Weise steuern zu können.

Insbesondere kann der der Filterfront zugeordnete Endbereich des das Filterelement in direktem Kontakt umschließenden Abschnitts ein die Kapillarität einengenden oder weitereinengenden Durchmesser aufweisen, sodaß mittels der so ausgestalteten Filterhülse und der entsprechenden Anordnung des Filterelements in der Filterhülse unmittelbar die Strömungsverhältnisse in optimaler Weise gesteuert werden können.

Weist die Vorrichtung wenigstens eine, mit wenigstens einer Kraftquelle koppelbare Einrichtung auf, so ermöglicht dies eine einfache, aber um so wirkungsvollerer Nutzung der Filterhülse durch die Möglichkeit einer einfachen Koppelung mit einer Kraftquelle.

Weist die Vorrichtung zwei, mit jeweils wenigstens einer Kraftquelle koppelbare Einrichtungen auf, so ermöglicht dies den Einsatz der Filterhülse unter Druckbeaufschlagung oder mittels Saugwirkung.

Sind die Einrichtungen mit dem Konus einer Spritze koppelbar und ist die Einrichtung ein mit der Filterhülse koppelbarer Hohlstopfen, so wird dadurch die Möglichkeit geschaffen, sich eine Kraftquelle zunutze zu machen, die sich als eine einfache Technik auszeichnet und ohne dies in Instituten für z.B. die medizinische Diagnostik vorhanden ist.

Weist der Hohlstopfen einen der Ankopplung dienenden Durchbruch auf, so wird eine Technik gewählt, die sich durch einen einfachen, aber um so wirkungsvolleren konstruktiven Aufbau auszeichnet.

Ist der Hohlstopfen wenigstens teilweise in die Filterhülse einschiebbar, festlegbar sowie lösbar ausgestaltet oder aber wenigstens teilweise über die Filterhülse schiebbar, festlegbar sowie lösbar ausgestaltet, so wird dadurch die Koppelung des Hohlstopfens mit der Filterhülse auf einfachste, aber um so praktikabelere Weise gewährleistet.

Es kann vorgesehen sein, daß der sich an den das Filterelement wenigstens teilweise in direktem Kontakt umschließenden Abschnitt anschließende, das Filterelement wenigstens teilweise im Abstand umhüllende Abschnitt sich direkt an den das Filterelement wenigstens teilweise in direktem Kontakt umschließenden Abschnitt anschließt.

Ist der das Filterelement in direktem Kontakt umschließende Abschnitt wenigstens teilweise zylinderförmig ausgestaltet oder ist der das Filterelement in direktem Kontakt umschließende Abschnitt wenigstens teilweise konisch zulaufend ausgestaltet, so wird dadurch die Möglichkeit geschaffen, je nach Bedarf, verschiedenartige Strömungsverhältnisse im Filterelement zu erreichen.

Ist das Filterelement mit seiner Filterfront wenigstens mit dem der Filterfront zugeordneten Ende der Filterhülse abschließend und die Filterfront freigebend in der Filterhülse angeordnet oder ist das Filterelement mit seiner Filterfront in einem Abstand aufpilzend aus der Filterhülse ragend in der Filterhülse angeordnet und festgelegt, so wird dadurch zum einen sichergestellt, daß die zu sammelnden und/oder abzutrennenden Partikel ohne Probleme an die Filterfront gelangen können und darüber hinaus wird sichergestellt, daß die Partikel durch einfaches Auftupfen auf z.B. einen Objektträger deponiert werden können.

Ist das Filterelement mit wenigstens einem, bei der Flüssigkeitsaufnahme, sich lösenden, an den Fasern gebundenen diagnostischen Farbstoff, insbesondere einem Vitalfarbstoff, versehen, so ermöglicht dies ohne separaten Arbeitsgang die Einfärbung der zu diagnostizierenden Partikel, da dieser Farbstoff bei entsprechender Benetzung in Lösung übergeht und die auf der Filterfront abgelagerten Partikel in herkömmlicher Art und Weise einfärbt.

Ist das Filterelement mit tumorspezifischen Antikörpern belegt, so kommt es ebenfalls ohne eines weiteren Arbeitsschrittes unmittelbar zu einer Kontaktierung der gesammelten und/oder abgetrennten Partikel mit den tumorspezifischen Antikörpern und damit zu den gewollten Reaktionen.

In der Zeichnung ist ein Ausführungsbeispiel der erfindungsgemäßen Vorrichtung zur Gewinnung und Wiedergewinnung von Zellen und Keimen aus Flüssigkeiten schematisch dargestellt, und zwar zeigt
- Fig. 1: eine perspektivische Seitenansicht der Vorrichtung,
- Fig.2: eine perspektivische Seitenansicht der Vorrichtung ohne den unteren Hohlstopfen,
- Fig.3: eine Draufsicht auf die Vorrichtung in Blickrichtung III aus Fig. 1 ohne den oberen Hohlstopfen,
- Fig.4: eine perspektivische Seitenansicht zweier gekoppelter Vorrichtungen,
- Fig.5: einen Schnitt nach Linie V-V aus Fig.2 ohne oberen Hohlstopfen,
- Fig.6: eine schematische Seitenansicht einer Spritze mit Probenflüssigkeit beinhaltendem Becher,
- Fig.7: eine perspektivische Seitenansicht der Vorrichtung mit in den oberen Hohlstopfen eingesteckter Spritze,
- Fig.8: eine Seitenansicht der Vorrichtung nebst in den oberen Hohlstopfen eingesteckter Spritze und in der Probenflüssigkeit eingetauchter Filterfront,
- Fig.9: eine perspektivische Seitenansicht der Vorrichtung mit in den oberen Hohlstopfen eingesteckter Spritze und auf einem Objektträger aufgesetzter Filterfront,
- Fig.10: eine perspektivische Seitenansicht der Vorrichtung ohne unteren Hohlstopfen, eingesteckt in ein Probenröhrchen,
- Fig.11: eine perspektivische Seitenansicht der Vorrichtung ohne unteren Hohlstopfen, eingesteckt in ein Probenröhrchen sowie mit dem gegenüberliegenden Ende eingesteckt in ein Reagenzröhrchen,

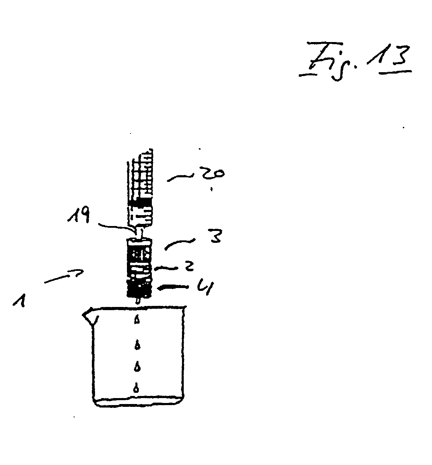

- Fig.12: eine perspektivische Seitenansicht der Vorrichtung nebst auf einem Objektträger aufgesetzter Filterfront und
- Fig.13: eine perspektivische Seitenansicht der Vorrichtung mit in den unteren Hohlstopfen eingesteckter Spritze und Flüssigkeitsauffangbehälter.

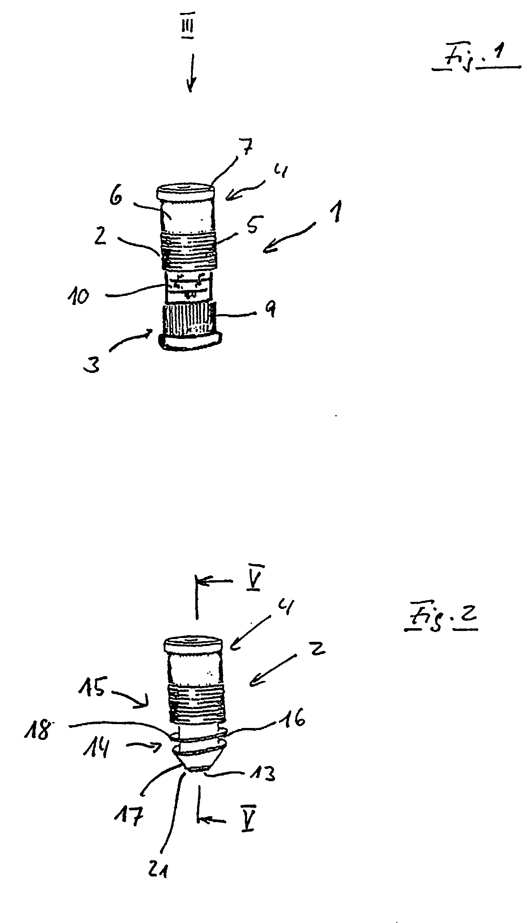

Wie der Fig.1 zu entnehmen, weist die erfindungsgemäße Vorrichtung (1) bzw. Patrone (1) eine Filterhülse (2) sowie einen unteren Hohlstopfen (3) und oberen Hohlstopfen (4) auf.

Der untere und obere Hohlstopfen (3,4) ist auf bzw. in die Filterhülse (2) einsteckbar und damit in und außer Wirkungseingriff bringbar ausgestaltet.

Im hier vorliegenden Ausführungsbeispiel ist, in der Zeichnung nicht erkennbar, die Filterhülse (2) rot ausgestaltet und mit einer umlaufenden, der besseren Handhabung dienenden Riffelung (5) versehen.

Der obere Hohlstopfen (4) ist im hier vorliegenden Ausführungsbeispiel weiß bzw. in seiner Umfangsfläche (6) milchig ausgestaltet und an seinem oberen, eine Lochplatte (7) mit einen mittig angeordneten Durchbruch (8) aufweisenden Ende durchsichtig, d.h. aus einem durchsichtigen Kunststoff wie z.B. Polypropylen.

An seinem unteren Ende weist die Vorrichtung (1) ebenfalls einen Hohlstopfen (3) auf, der zum einen einen der Handhabung des Stopfens (3) dienenden Rändelbereich (9) aufweist, sowie einen darüber angeordneten und trotz der insgesamt blauen Ausgestaltung des Stopfens weitgehend durchsichtigen Kontaktabschnitt (10), der der Festlegung des Hohlstopfens (3) auf der Filterhülse (2) dient.

An seinen unteren Ende ist der Hohlstopfen (3) darüber hinaus mit einer Lochplatte (11) versehen, die mittig einen Durchbruch (12) aufweist.

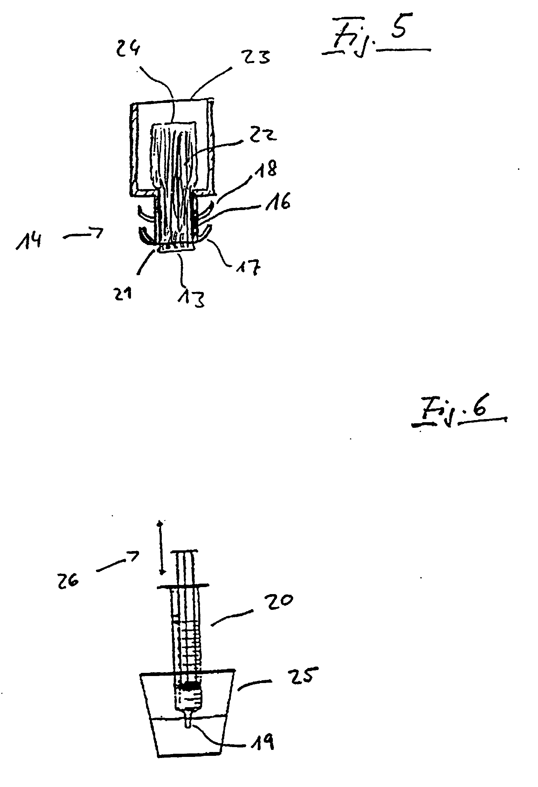

Wie der Fig.2 zu entnehmen, weist die Filterhülse (2) in ihrem unteren Bereich, d.h. dem, sich an die Filterfront (13) anschließenden Abschnitt (14) einen geringeren Außen- und, wie Fig.5 zeigt auch einen geringeren Innendurchmesser als in dem sich daran anschließenden die Riffelung (5) aufweisenden Abschnitt (15) der Filterhülse (2) auf.

Auf der Außenwandung (16) des, sich an die Filterfront (13) anschließenden Abschnitts (14) der Filterhülse (2) sind, im hier vorliegenden Ausführungsbeispiel, zwei Dichtlippen (17, 18) wie aus Figuren 2 und 5 zu ersehen angeordnet, die der Festlegung des unteren Hohlstopfens (3) dienen.

Diese Dichtlippen (17,18) sind im hier vorliegenden Ausführungsbeispiel in einem Abstand von 4 mm zueinander angeordnet und derart ausgedünnt gestaltet, daß eine gewollte Flexibilität vorhanden ist, die einerseits ein Aufschieben des unteren Hohlstopfens (3) über die Dichtlippen (17, 18) ermöglicht, ebenso wie ein Abziehen des unteren Hohlstopfens (3) und gleichzeitig dafür Sorge trägt, daß eine absolute Flüssigkeitsdichtigkeit gegen die Innenwandung des unteren Hohlstopfens (3) gewährleistet ist.

Die in den Hohlstopfen (3,4) bzw. den Lochplatten (7,11) vorgesehenen Durchbrüche (8,12) sind hinsichtlich ihres Durchmessers so angelegt, daß jeweils der Konus (19) dargestellt in Figuren 7, 8 und 6 einer Rekord-Spritze (20) bündig und flüssigkeitsdicht eingeschoben werden kann.

In einem weiteren, hier nicht dargestellten Ausführungsbeispiel, können diese Öffnungen (8,12) mit einem Adapter versehen werden, so daß auch andere, als die zuvor beschriebenen Spritzentype mit den Hohlstopfen (3,4) und damit der Vorrichtung (1) gekoppelt werden können, wie z.B. eine Spritze mit einem Luer-Lok-Konus.

Im hier vorliegenden Ausführungsbeispiel ist die Filterhülse (2) 20 mm lang ausgestaltet, wobei der obere Abschnitt (15), d.h. der die Riffelung (5) aufweisende Abschnitt (15) auf einer Strecke von 9 mm einen Innendurchmesser von 12 mm aufweist und der sich daran anschließende untere Abschnitt (14), im hier vorliegenden Ausführungsbeispiel eine Länge von 10 mm und einen Innendurchmesser von 7,5 mm aufweist, wobei seine Endöffnung (21), d.h. die der Filterfront (13) zugewandte Öffnung (21) variabel, je nach gewünschtem Einsatz ausgebildet wird.

In der Filterhülse (2) ist ein Filterrundstäbchen (22), wie aus Figuren 3 und 5 zu ersehen, festgelegt, wobei dieses Filterrundstäbchen (22) der jeweiligen Aufgabe entsprechend angepaßt wird und aus vergrößertem 2 ½ Acetat- oder Polyesterfasern hergestellt ist.

Das Filterrundstäbchen (22) weist ein längsgerichtetes Fasermaterial mit unterschiedlichen Titern auf, das einheitlich mit Triacetin verpreßt für unterschiedliche technische Anwendung zur Verfügung steht.

Dieses an sich bekannte Filterrundstäbchen (22) wurde für den hier vorliegenden Einsatz, inbesondere dem Einsatz in der Filterhülse (2), einer speziellen Behandlung unterzogen, d.h. es wurde dafür Sorge getragen, daß eine einstellbare Kapillarität im Mikrobereich erreicht wird und inbesondere dadurch, daß partielle Kapillaritätsverdichtungen vorgenommen wurden.

Es können Filterrundstäbchen (22) mit unterschiedlichen Kapillargrößen und Längen in angepaßte Patronen (1) bzw. Filterhülsen (2) flüssigkeitsdicht aneinander gekoppelt werden.

Wie der Fig.5 zu entnehmen, weist die Endöffnung (21) einen geringeren Durchmesser als der sich unmittelbar daran anschließenden Innendurchmesser des unteren Abschnittes (14) auf, so daß durch die dadurch entstehende einengende Wirkung die Frontkapillarität des Filterstäbchens (22) im Bereich der Filterfront (13) bewußt verändert wird.

Wenn eine Filterfront (13) Kapillaröffnungen von 10µm aufweist, werden die Zellen, die über diesem Durchmesser liegen, wie z.B. Plattenepithelzellen aus einer Flüssigkeit quantitativ abgeschieden, bzw. dort angelagert, während kleinere Zellen, wie z.B. Lymphozyten, kleine Tumorzellen oder Blutzellen das Filter passieren und der Diagnostik verloren gehen. Kleine Partikel bzw. Keime unter 2µm werden dabei gar nicht oder nur als Auflagerungen auf größeren Zellen erreicht.

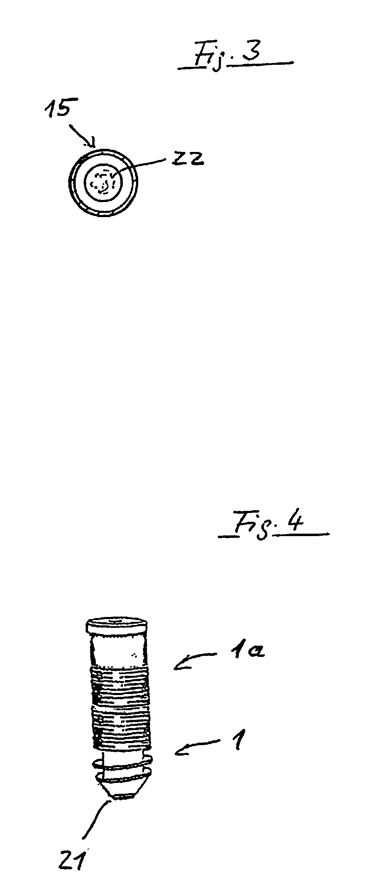

Aus diesem Grund ist es, wie in Fig.4 dargestellt möglich, verschiedene Vorrichtungen (1, 1a) derart miteinander zu koppeln, daß der die Dichtlippen aufweisende Teil (14) einer Vorrichtung (1a) in z.B. das obere Ende (23) einer Filterhülse (2) derart eingeführt wird, daß die Dichtlippen (17,18) mit der Innenwandung des oberen Teils (15) der Filterhülse (2) in Wirkungseingriff gelangen, d.h. die Filterhülse (2) in diesen Abschnitt unter Dichteschluß zu plazieren ist und die Filterfront (13) flüssigkeitsdicht mit dem oberen Ende (24) des Filterrundstäbchens(22) in Wirkungseingriff gelangt.

Um die Frontkapillarität jedoch nicht nur durch die Wahl verschieden ausgestalteter Filterhülsen (2) beeinflussen zu können, ist es in einem weiteren Ausführungsbeispiel denkbar die Endöffnung (21) nach Art eines Bohrfutters auszugestalten, so daß der Durchmesser der Endöffnung (21) variabel ausgebildet werden kann.

Dabei wäre es denkbar, den durch die untere Dichtlippe (17) konisch ausgestalteten Tubusteil (14) im Bereich der Austrittsöffnung (21) z.B. mit drei Schlitzen zu versehen und an der Außenwandung umlaufende Gewindegänge, d.h. Schraubrillen vorzusehen.

Mittels einer entsprechend ausgebildeten Mutter könnte dann der Enddurchmesser der Endöffnung (21) variabel, d.h. stufenlos verändert werden und damit auch die Frontkapillarität.

Es wäre ebenso denkbar, diesen Bereich der Austrittsöffnung (21) mit drei Schlitzen zu versehen und eine Aufsatzhülse mit ausgewähltem Innendurchmesser vorzusehen, so daß dann mittels dieser Aufsatzhülse durch einfaches verschieben, der Konizität folgend die geschlitzten Öffnungsteile zusammengepreßt werden, so daß sich dadurch der Durchmesser der Endöffnung (21) bzw. Durchmesser des Endöffnungsbereichs (14) ansteuern läßt und variabel veränderbar ausgestaltet ist.

Der obere Hohlstopfen (4) wird nicht, wie der untere Hohlstopfen über am Außendurchmesser (16) des Filterhülsenabschnitts (14) angeordnete Dichtlippen in Wirkungseingriff mit der Filterhülse (2) gebracht, sonderen vielmehr greift der obere Hohlstopfen (4) in die Filterhülse (2) ein, d.h. der Hohlstopfen (4) ist flüssigkeitsdicht in der Filterhülse (2) festzulegen und stülpt sich dabei über den rückwärtigen Teil (24) des in der Filterhülse (2) festgelegten Filterrundstäbchens (22).

Nachstehend wird die Funktionsweise der Vorrichtung anhand von Anwendungsbeispielen beschrieben:

Sollen aus einer wäßrigen Körperflüssigkeit, wie z.B. Urin, die darin befindlichen und von der Blasenwand abgeschilferten Zellen repräsentativ gewonnen werden und der mikroskopischen Diagnostik zugeführt werden, ist es möglich die in einem großlumigen Gefäß (25) (ca. 100 ml fassend) gesammelte Urinprobe sofort dem Gewinnungsprozeß zuzuführen oder bei sehr zellarmen Urinen die Proben vorbereitend einige Stunden bei 4° C ruhen zu lassen. Dabei sinken alle zellulären Schwebteilchen auf den Gefäßboden ab und können von diesem quantitativ aufgesaugt werden.

Die Vorrichtung (1) kann für die Zellgewinnung aus Urin oder aus Blasenflüssigkeit vorteilhaft nach dem sogenannten Durchlaufprinzip eingesetzt werden.

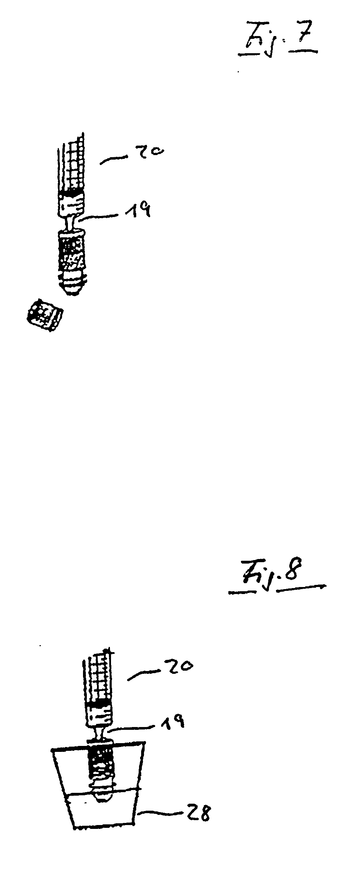

Bei diesem Durchlaufprinzip wird, entsprechend Fig.6, mittels einer 10 ml Rekordspritze (20) die Probenflüssigkeit durch mehrmaliges Aufziehen und Auspressen gemäß Pfeil (26) durchmischt, so daß alle sich darin befindlichen Teilchen gleichmäßig in der Flüssigkeit verteilt sind.

Dann wird die Spritzenfüllung aspiriert.

Entsprechend eines weiteren Ausführungsbeispieles, d.h. bei z.B. vorliegenden zellarmen Urinen wird das durch entsprechende Ruhephasen auf dem Gefäßboden sedimentierte Zellmaterial direkt, d.h. ohne vorherige Durchmischung mittels der Spritze aspiriert.

Nach dem Aspirationsvorgang wird auf den Konus (19) der gefüllten Spritze (20) die Vorrichtung (1) bzw. Patrone (1) aufgesteckt, wobei das die Filterfront (13) aufweisende Ende der Patrone (1), d.h. das den unteren Hohlstopfen (3) aufweisende Ende der Patrone (1) über die in der Lochplatte (11) angeordnete Bohrung (12) mit dem Konus (19) der Spritze (20) in Wirkungseingriff gebracht wird, so daß der Konus (19) auf die Filterfront (13) gerichtet ist.

Da, wie bereits dargelegt, die Hohlstopfen (3,4) unterschiedlich farbig markiert sind, d.h. der untere Hohlstopfen (3) blau und der oberen Hohlstopfen (4) weiß ausgestaltet ist, ist bei der Handhabung auch eine optische Erkennbarkeit möglich, ohne daß jeweils der Hohlstopfen (3,4) von der Filterhülse (2) entfernt werden muß, um eine entsprechende Orientierung hinsichtlich der Filterfrontseite (13) vornehmen zu müssen.

Der Filtrationsvorgang wird durch Absenken des Spritzenkolbens durchgeführt, d.h. die Probenflüssigkeit wird durch Absenken des Kolbens langsam durch das Filter (13,22) bzw. das Filterrundstäbchen (22) durchgeführt, wobei die Spritze (22) mit der aufgesteckten Patrone (1) entsprechend Fig.13 senkrecht gehalten wird, d.h. die Vorrichtung (1) befindet sich unterhalb der Spritze (20).

Der Absenkungsvorgang des Kolbens erfolgt zügig, aber nicht schneller als ein üblicher Injektionsvorgang.

Bei diesem Vorgang sammeln sich alle Zellen, die größer sind als die Kapillaren des Filterstäbchens (22) bzw. der Filterfront (13) auf der Frontfläche (13) des Filters (22). Die zellfreie Flüssigkeit tropft aus der öffnung (8) des rückwärtigen d.h. oberen Hohlstopfens (4) und wird verworfen.

Wenn der Filtrationsvorgang beendet ist, wird die Patrone (1) von der Spritze (20) abgezogen und in umgedrehter Position mit der Öffnung (12) des unteren Hohlstopfens (3), d.h. dem der Filterfront (13) zugeordneten Hohlstopfens (3) auf den Spritzenkonus (19) aufgesteckt.

In dieser Position, d.h. mit nach oben gerichteter Patrone (1) wird durch Betätigung des Spritzenkolbens eventuell auf der Filterfront (13) befindliche Überschußflüssigkeit nach hinten weg gesogen, d.h. z.B. in den hinteren Bereich des Filterrundstäbchens (22), d.h. dem der Filterfront (13) abgewandten Bereich (24).

Bei angekoppelter Spritze wird nun der untere Hohlstopfen (3), d.h. der der Filterfront (13) zugeordnete Hohlstopfen (3) von der Filterhülse (2) abgezogen.

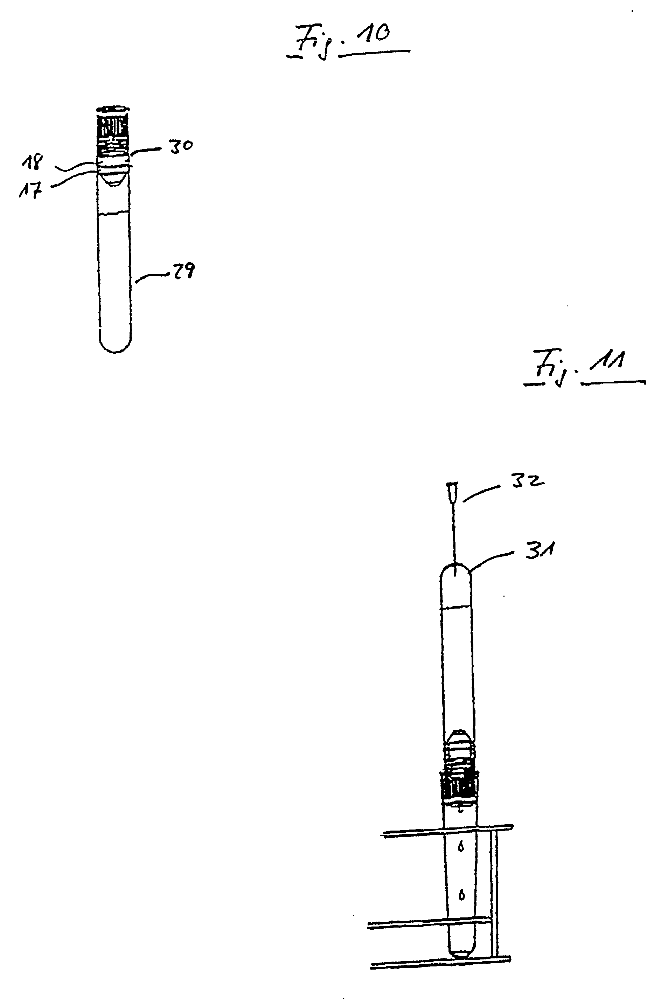

Die Zellübertragung erfolgt in dem die nun freiliegende Filterfront (13) auf einen Objektträger (27), dargestellt in Fig.9, abgedrückt wird.

Auf einer Objektträgerfläche lassen sich dabei bis zu fünf Abdrücke deponieren.

Wurde viel Material abgeschieden, lassen sich weitere Flächen belegen.

Bei diesem Vorgang hat die angekoppelte Spritze (20) eine sehr vorteilhafte Funktion, weil durch die Betätigung des Spritzenkolbens die Feuchtigkeit in den Abdruckarealen beeinflußt bzw. reguliert werden kann.

Sollen aus einer Probe weitere Präparate hergestellt werden, ist die benutzte Patrone (1) insgesamt wenigstens dreimal einsetzbar.

Die so hergestellten Präparate werden nach dem Abtrocknen mittels der bekannten Fixierungs- und Färbverfahren weiterverarbeitet und der mikroskopischen Diagnostik zugeführt.

Die vorstehende beschriebene Systemanordnung hat die Aufgabe, aus Urin die typischen Urothelzellen, die von der Blasenwand abgeschilfert wurden, abzutrennen. Diese Zelltypen liegen mit ihrem Durchmesser überwiegend im Bereich von 12 µm. Für die Krebsdiagnostik nicht relevante Zellen, wie z.B. Leukozyten und Erytrozyten liegen in der Größe zwischen 7 und 8 µm und sollen in der Anzahl vom Präparat stark dezimiert vorhanden sein, weil eine Überpopulation dieser Typen das Erkennen wichtiger anderer Zellen erschweren kann.

Deshalb ist die Frontöffnung (21) der Filterpatrone (2) auf einen Durchmesser von 7 mm ausgelegt. Für das eingefügte Filterelement (22) ergibt sich dadurch eine Frontverdichtigung, die eine Kapillarität von 12 µm erzeugt.

Wenn die Diagnoseverfahren eine separate Abtrennung kleiner Zellen verlangen, wie z.B. Leukozyten, Lymphozyten und kleinerer Tumorzellen, so ist dieser Vorgang dem erfindungsgemäßen Gedanken folgend einfach und effektiv erreichbar.

Eine zweite Patrone (1a) ist mit einer Frontöffnung (21) von 6 mm Durchmesser versehen, so daß das eingeschobene Filterelement (22) an der Front (13) stärker eingeengt ist und eine Kapillarität von 8 µm aufweist.

Es ist verfahrenstechnisch vorgesehen, daß die Patronen in Reihe, wie in Fig.4 dargestellt, durch rückwärtiges Einstecken, koppelbar sind.

Nach dem der Filtrationsvorgang mittels einer Doppelpatrone (1, 1a) durchgeführt wurde, werden diese zum Zwecke einer Zellwiedergewinnung voneinander getrennt und auf gekennzeichnete Objektträger separiert abgedrückt.

Eine dritte ankoppelbare Patrone (1b) weist einen Frontdurchmesser, d.h. einen Durchmesser der Endöffnung (21) von 4 mm auf und erreicht eine Kapillarität, die geeignet ist, auch Keime in separater Schichtung zurückzuerhalten.

Im Anwendungsversuch gelang es von der Filterfront (13) des Filters (22) eine Direktübertragung auf Agar oder Nährmedien, so daß alle gängigen mikrobiologischen Untersuchungen durchgeführt werden konnten.

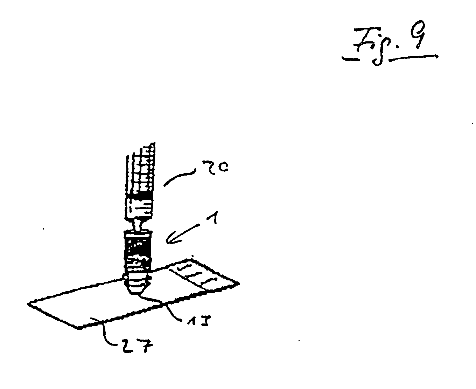

Wenn in einem großlumigen Probengefäß (28) nur wenige mm Untersuchungsflüssigkeit zur Verfügung stehen, kann diese Flüssigkeit direkt mittels der an einer Spritze (20) angekoppelten Filterpatrone (4,2) aspiriert werden. Hierbei wird der untere Hohlstopfen (3) von der Filterhülse (2) entfernt, so daß die Filterfront (13) freiliegt. Die Spritze wird in den rückwärtigen Hohlstopfen (4), d.h. den oberen Hohlstopfen (4) eingesteckt. Die Filterfront (13) wird beim Gewinnungsvorgang in die Flüssigkeit eingetaucht, so daß die Flüssigkeit komplett in die Spritze eingesaugt werden kann (siehe Fig.8).

Bei der Übertragung des, der Filterfront (13) anhaftenden Zellmaterials auf einen Objektträger (27) verbleibt das zellfreie Aspirat im Spritzenkörper (20).

Die Saugfunktion der Vorrichtung (1) kann auch dann vorteilhaft eingesetzt werden, wenn Proben in speziellen Reagenzröhrchen (29) zur Verfügung stehen. Diese Röhrchen (29) werden vorzugsweise aus Polyäthylen hergestellt und sind im oberen Öffnungsdurchmesser (30) dem äußeren Durchmesser der Filterhülse (2), insbesondere dem der Dichtlippen (17,18) angepaßt, so daß die Filterhülse (2) flüssigkeitsdicht in die Röhrchenöffnung (30) eingeführt werden kann.

Auch hier übernehmen die Dichtlippen (17, 18) im unteren Abschnitt (14) der Filterhülse (2) eine abdichtende Funktion.

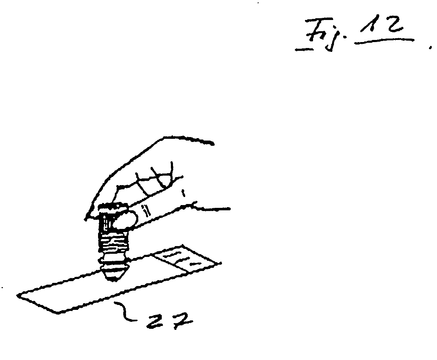

Wenn die Spritze (20) auf den rückwärtigen, d.h. oberen Hohlstopfen (4) der Patrone (1) aufgesetzt ist, kann die zusammengekoppelte Einheit umgedreht werden. Bei den aus Weichplastik hergestellten Röhrchen (29) wird nun am geschlossenen Pohl (31), d.h. dem der Patrone (1) abgewandten Ende des Röhrchens (29) eine Kanüle (32) entsprechend Fig. 11 eingestochen. Aus dem so belüfteten System kann die gesamte Flüssigkeit durch die Filterpatrone (1) über die Filterfront (13) in den Spritzenkörper (20) eingesaugt werden. Die Zellübertragung erfolgt wie zuvor beschrieben.

Vorteilhafter Weise ist dabei das Probenröhrchen (29) in seiner Ausgestaltung der erfindungsgemäßen Vorrichtung (1) angepaßt, in dem auch der untere Pohl (31), d.h. daß der Vorrichtung (1) abgewandte Ende des Röhrchens (29) offen ausgebildet und mittels eines Stopfens oder einer Belüftungsschraube verschließbar ist.

Die zuvor beschriebene und notwendige Belüftung kann dann durch Entfernen oder Aufdrehen dieses Verschlusses bzw. Betätigen des Lüftungsventils erfolgen.

Für den Versand einer Probe kann ein derart beidseitig verschlossenes Röhrchen (29) ohne Einschränkung verwendet werden.

Anwendungsversuche haben gezeigt, daß die Vorrichtung (1) auch für eine Durchlauffiltration sehr effektiv einsetzbar ist.

Entsprechend eines Beispieles wurden die Liquorproben untersucht, die in unterschiedlichen Flüssigkeitsmengen zwischen 1 und 6 ml zur Verfügung standen.

Dabei ist es bekannt, daß die sich in der Rückenmarkflüssigkeit befindlichen Zellen nach dem Punktionsvorgang, wobei das Material in Probenröhrchen überführt wird, sehr schnell lysieren und durch mechanische Einflüsse leicht verändert oder zerstört werden können. Geschädigte Zellen verlieren sehr schnell ihre typischen feinmorphologischen Merkmale und lassen sich gar nicht oder nur eingeschränkt mikroskopisch beurteilen.

Deshalb wurde für die Zellgewinnung aus Liquor, unter Anwendung der erfindungsgemäßen Vorrichtung (1) die Funktion der Durchlauffiltration gewählt.

Die in einem angepaßten Röhrchen (29) präparierte Probe wird nach dem Eingang kurz bei 4° C gelagert.

In das entstöpselte Röhrchen (29) wird die Filterpatrone (4,2), entsprechend Fig.10 mit der offenen Filterfront (13) gegen die Probenflüssigkeit gerichtet, eingeschoben. An der Rückseite der Filterhülse (2) verbleibt der obere Hohlstopfen (4) in seiner ursprünglichen Position.

Das mit der Patrone (1) bestückte Probenröhrchen (29) wird in umgedrehter Richtung, dargestellt in Fig.11 in ein weitlumigeres konisches Röhrchen (33) eingefügt bzw. im oberen Drittel dieses Röhrchens (33) so positioniert, daß sich die Patrone (1) im konischen Verlauf festsetzt, aber seitlich noch Luft durchläßt.

Nun wird das Probenröhrchen (29) wie zuvor beschrieben, mittels Kanüle (32) oder bei modifizierten Probenröhrchen (29) durch Belüftung mittels Ventil belüftet.

Je nach Viskosität der Flüssigkeit läuft diese zügig oder auch langsam durch die Filterpatrone (4,2) d.h. durch die Filterfront (13) und das Filterrundstäbchen (22), wobei sich die Zellen auf der Filterfront (13) ablagern und die filtrierte Flüssigkeit nach unten in das Auffanggefäß (33) durch den oberen Hohlstopfen (4) bzw. die darin angeordnete Bohrung (8) nach unten in das Auffangsgefäß (33), d.h. das Reagenzglas (33) läuft.

Dem Abfließstrom folgend sedimentieren die Zellen auch ohne Provokation auf der Filterfläche (13).

Bei hochviskosen Liquorproben oder auch bei zellreichem Inhalt kann der Abfluß stagnieren oder ganz aufhören.

Der Filterationsvorgang ist damit beendet und die gesamte Einheit wird vom Auffanggefäß, d.h. Reagenzglas (33) getrennt und langsam umgedreht, so daß die Einheit eine Stellung ensprechend Fig.10 einnimmt.

In den Durchbruch bzw. die Bohrung (8) des oberen Hohlstopfens (4) wird eine Spritze (20) eingesteckt und der Kolben, im hier vorliegenden Ausführungsbeispiel bis zur Marke 1 ml angezogen.

Anschließend wird die Vorrichtung (1) mit angekoppelter Spritze (20) vom Probengefäß (29) getrennt.

Der Kolben der Spritze (20) wird noch einmal betätigt, um überschüssige Flüssigkeit von der Filterfront (13) zu entfernen. Dann werden die Zellen von der Filterfront (13) legeartis auf Objektträger (27) übertragen.

## Patentansprüche

1. Vorrichtung für das Sammeln und Abtrennen von Partikeln aus einer Flüssigkeit für die medizinische Diagnose und/oder technische Filtration, aufweisend ein von einer beiderends offenen Filterhülse (2) festgelegtes Filterelement (22), wobei das Filterelement (22) von einem ersten Abschnitt (14) der Filterhülse (2) wenigstens teilweise in direktem Kontakt umschlossen und von einem daran anschließenden zweiten Abschnitt (15) der Filterhülse (2) mit Abstand umhüllt ist, und wobei der erste Abschnitt (14) einen geringeren Außendurchmesser als der daran anschließende zweite Abschnitt (15) und wenigstens eine umlaufende Dichtlippe (17,18) aufweist für ein Ankoppeln an einen Hohlstopfen, ein Reagenzröhrchen und/oder an den ein Filterelement mit Abstand umhüllenden Abschnitt einer weiteren Filterhülse.

2. Vorrichtung nach Anspruch 1, **dadurch gekennzeichnet, dass** zwei Dichtlippen (17,18) vorgesehen sind.

3. Vorrichtung nach einem oder mehreren der vorangehenden Ansprüche, **dadurch gekennzeichnet, dass** wenigstens eine die Kapillarität des Filterelements (22) wenigstens partiell verändernde Einrichtung (21) vorgesehen ist.

4. Vorrichtung nach einem oder mehreren der vorangehenden Ansprüche, **dadurch gekennzeichnet, dass** zwei die Kapillarität des Filterelements (22) wenigstens partiell verändernde Einrichtungen (21) vorgesehen sind.

5. Vorrichtung nach einem oder mehreren der vorangehenden Ansprüche, **dadurch gekennzeichnet, dass** die Einrichtung (21) im Bereich (14) der Filterfront (13) vorgesehen ist.

6. Vorrichtung nach einem oder mehreren der vorangehenden Ansprüche, **dadurch gekennzeichnet, dass** der der Filterfront (13) zugeordnete Endbereich (21) des das Filterelement (22) in direktem Kontakt umschließenden Bereichs (14) ein die Kapillarität einengenden Durchmesser aufweist.

7. Vorrichtung nach einem oder mehreren der vorangehenden Ansprüche, **dadurch gekennzeichnet, dass** die Filterhülse (2) wenigstens eine mit wenigstens einer Kraftquelle (20) koppelbare Einrichtung (3;4) aufweist.

8. Vorrichtung nach einem oder mehreren der vorangehenden Ansprüche, **dadurch gekennzeichnet, dass** die Einrichtungen (3;4) mit dem Konus (19) einer Spritze (20) koppelbar ausgestaltet sind.

9. Vorrichtung nach einem oder mehreren der vorangehenden Ansprüche, **dadurch gekennzeichnet, dass** die Einrichtung ein mit der Filteraufnahme (2) koppelbarer Hohlstopfen (3;4) ist.

10. Vorrichtung nach einem oder mehreren der vorangehenden Ansprüche, **dadurch gekennzeichnet, dass** der Hohlstopfen (3;4) ein der Ankopplung dienenden Durchbruch (8;12) aufweist.

11. Vorrichtung nach einem oder mehreren der vorangehenden Ansprüche, **dadurch gekennzeichnet, dass** ein Hohlstopfen (3) wenigstens teilweise über die Filterhülse (2;14) schiebbar, festlegbar sowie lösbar, ausgestaltet ist.

12. Vorrichtung nach einem oder mehreren der vorangehenden Ansprüche, **dadurch gekennzeichnet, dass** der Hohlstopfen (4) wenigstens teilweise in die Filterhülse (2;15) einschiebbar, festlegbar sowie lösbar, ausgestaltet ist.

13. Vorrichtung nach einem oder mehreren der vorangehenden Ansprüche, **dadurch gekennzeichnet, dass** der sich an den das Filterelement (22) wenigstens teilweise in direktem Kontakt umschließenden Abschnitt (14) anschließende, das Filterelement (22) wenigstens teilweise im Abstand umhüllende Bereich (15) sich direkt anschließt.

14. Vorrichtung nach einem oder mehreren der vorangehenden Ansprüche, **dadurch gekennzeichnet, dass** der das Filterelement (22) in direktem Kontakt umschließende Bereich (14) wenigstens teilweise zylinderförmig ausgestaltet ist.

15. Vorrichtung nach einem oder mehreren der vorangehenden Ansprüche, **dadurch gekennzeichnet, dass** der das Filterelement (22) in direktem Kontakt umschließende Bereich (14) wenigstens teilweise konisch zulaufend ausgestaltet ist.

16. Vorrichtung nach einem oder mehreren der vorangehenden Ansprüche, **dadurch gekennzeichnet, dass** das Filterelement (22) mit seiner Filterfront (13) wenigstens mit dem der Filterfront (13) zugeordneten Ende (21) der Filterhülse (2) abschließend und die Filterfront (13) freigebend in der Filterhülse (2) angeordnet ist.

17. Vorrichtung nach einem oder mehreren der vorangehenden Ansprüche, **dadurch gekennzeichnet, dass** das Filterelement (22) mit seiner Filterfront (13) in einem Abstand aufpilzend aus der Filterhülse (2) ragend in der Filterhülse (2) angeordnet und festgelegt ist.

18. Vorrichtung nach einem oder mehreren der vorangehenden Ansprüche, **dadurch gekennzeichnet, dass** das Filterelement (22) mit wenigstens bei der Flüssigkeitsaufnahme sich lösenden an den Fasern gebundenen diagnostischen Farbstoff, insbesondere Vitalfarbstoff versehen ist.

19. Vorrichtung nach einem oder mehreren der vorangehenden Ansprüche, **dadurch gekennzeichnet, dass** das Filterelement (22) mit tumorspezifischen Antikörpern belegt ist.

## Claims

1. A device for collecting and separating particles from a fluid for the purpose of medical diagnosis and/or technical filtration, having a filter element (22) fixed by a filter sleeve (2) open at both ends, whereby the filter element (22) is enclosed at least partially in direct contact by a first section (14) of the filter sleeve (2) and is sheathed with clearance by a second connecting section (15) of the filter sleeve (2), and whereby the first section (14) has a lesser external diameter than the second section (15) connected thereto and has at least one circular sealing lip (17, 18) for coupling to a hollow stopper, a reaction tube and/or to the section of another filter sleeve sheathing a filter element with clearance.

2. The device as claimed in Claim 1, **characterised in that** two sealing lips (17, 18) are provided.

3. The device as claimed in any one or more of the foregoing claims, **characterised in that** at least one mechanism (21) is provided at least partially altering the capillarity of the filter element (22).

4. The device as claimed in any one or more of the foregoing claims, **characterised in that** two mechanisms (21) are provided at least partially altering the capillarity of the filter element (22).

5. The device as claimed in any one or more of the foregoing claims, **characterised in that** the mechanism (21) is provided in the region (14) of the filter face (13).

6. The device as claimed in any one or more of the foregoing claims, **characterised in that** the end region (21) of the region (14) enclosing the filter element (22) in direct contact assigned to the filter face (13) has a diameter constricting the capillarity.

7. The device as claimed in any one or more of the foregoing claims, **characterised in that** the filter sleeve (2) has at least one mechanism (3; 4) which can be coupled to at least one power source (20).

8. The device as claimed in any one or more of the foregoing claims, **characterised in that** the mechanisms (3; 4) are designed to couple to the cone (19) of a syringe (20).

9. The device as claimed in any one or more of the foregoing claims, **characterised in that** the mechanism is a hollow stopper (3; 4) which can be coupled to the filter uptake (2).

10. The device as claimed in any one or more of the foregoing claims, **characterised in that** the hollow stopper (3; 4) has an opening (8; 12) serving as coupling means.

11. The device as claimed in any one or more of the foregoing claims, **characterised in that** a hollow stopper (3) is designed to be at least partially pushed, fixed and detached by way of the filter sleeve (2; 14).

12. The device as claimed in any one or more of the foregoing claims, **characterised in that** the hollow stopper (4) is designed to be pushed, fixed and detached at least partially in the filter sleeve (2; 15).

13. The device as claimed in any one or more of the foregoing claims, **characterised in that** the region (15) connecting to the section (14) enclosing the filter element (22) at least partially in direct contact and enclosing the filter element (22) is connected directly at least partially at a distance.

14. The device as claimed in any one or more of the foregoing claims, **characterised in that** the region (14) enclosing the filter element (22) in direct contact is designed at least partially cylindrical.

15. The device as claimed in any one or more of the foregoing claims, **characterised in that** the region (14) enclosing the filter element (22) in direct contact is designed at least partially conically.

16. The device as claimed in any one or more of the foregoing claims, **characterised in that** the filter element (22) with its filter face (13) is arranged at least terminating with the end (21) of the filter sleeve (2) assigned to the filter face (13) and the filter face (13) is arranged to release in the filter holder (2).

17. The device as claimed in any one or more of the foregoing claims, **characterised in that** the filter element (22) is arranged and fixed with its filter face (13) at a distance mushrooming out of the filter sleeve (2) projecting in the filter sleeve (2).

18. The device as claimed in any one or more of the foregoing claims, **characterised in that** the filter element (22) is provided with diagnostic dye, in particular vital dye, bound to the fibres dissolving at least during fluid uptake.

19. The device as claimed in any one or more of the foregoing claims, **characterised in that** the filter element (22) is coated with tumor-specific antibodies.

## Revendications

1. Dispositif pour collecter et séparer des particules d'un liquide à des fins de diagnostic médical et/ou filtration technique, présentant un élément filtrant (22) maintenu par un manchon de filtre (2) ouvert des deux côtés, l'élément filtrant (22) étant entouré au moins partiellement en contact direct par une première section (14) du manchon du filtre (2) et enveloppé avec un écartement par une deuxième section (15) s'y raccordant du manchon du filtre (2) et la première section (14) présentant un diamètre extérieur inférieur à celui de la deuxième section (15) s'y raccordant et au moins une lèvre d'étanchéité périphérique (17, 18) pour accouplement à un bouchon creux, un petit tube de réactif et/ou à une section enveloppant avec un écartement un élément filtrant d'un autre manchon de filtre.

2. Dispositif selon la revendication 1, **caractérisé en ce que** deux lèvres d'étanchéité (17, 18) sont prévues.

3. Dispositif selon une ou plusieurs des revendications précédentes, **caractérisé en ce qu'**au moins un dispositif (21) modifiant au moins partiellement la capillarité de l'élément filtrant (22) est prévu.

4. Dispositif selon une ou plusieurs des revendications précédentes, **caractérisé en ce que** deux dispositifs (21) modifiant au moins partiellement la capillarité de l'élément filtrant (22) sont prévus.

5. Dispositif selon une où plusieurs des revendications précédentes, **caractérisé en ce que** le dispositif (21) est prévu dans la zone (14) de l'avant du filtre (13).

6. Dispositif selon une ou plusieurs des revendications précédentes, **caractérisé en ce que** la zone d'extrémité (21) associée à l'avant du filtre (13) de la zone (14) entourant en contact direct l'élément filtrant (22) présente un diamètre restreignant la capillarité.

7. Dispositif selon une ou plusieurs des revendications précédentes, **caractérisé en ce que** le manchon du filtre (2) présente au moins un dispositif (3; 4) pouvant être couplé avec au moins une source d'énergie (20).

8. Dispositif selon une ou plusieurs des revendications précédentes, **caractérisé en ce que** les dispositifs (3; 4) sont conçus de manière à pouvoir être couplés avec le cône (19) d'une seringue (20).

9. Dispositif selon une ou plusieurs des revendications précédentes, **caractérisé en ce que** le dispositif est un bouchon creux (3; 4) pouvant être couplé avec le récepteur du filtre (2).

10. Dispositif selon une ou plusieurs des revendications précédentes, **caractérisé en ce que** le bouchon creux (3; 4) présente une percée (8; 12) servant à l'accouplement.

11. Dispositif selon une ou plusieurs des revendications précédentes, **caractérisé en ce qu'**un bouchon creux (3) est conçu de manière à pouvoir être poussé, fixé aussi bien que détaché sur le manchon du filtre (2; 14).

12. Dispositif selon une ou plusieurs des revendications précédentes, **caractérisé en ce que** le bouchon creux (4) est conçu de manière à pouvoir être poussé, fixé aussi bien que détaché au moins partiellement dans le manchon.du filtre (2; 15).

13. Dispositif selon une ou plusieurs des revendications précédentes, **caractérisé en ce que** la zone (15) se raccordant à la section (14) entourant au moins partiellement en contact direct l'élément filtrant (22), enveloppant au moins partiellement avec un écartement l'élément filtrant (22) se raccorde directement.

14. Dispositif selon une ou plusieurs des revendications précédentes, **caractérisé en ce que** la zone (14) entourant en contact direct l'élément filtrant (22) est configurée au moins partiellement en forme de cylindre.

15. Dispositif selon une ou plusieurs des revendications précédentes, **caractérisé en ce que** la zone (14) entourant en contact direct l'élément filtrant (22) est configurée au moins partiellement avec une orientation conique.

16. Dispositif selon une ou plusieurs des revendications précédentes, **caractérisé en ce que** l'élément filtrant (22) avec son avant de filtre (13) est disposé au moins de manière à se terminer au moins par l'extrémité (21) associée à l'avant de filtre (13) du manchon de filtre (2) et à libérer l'avant du filtre (13) dans le manchon du filtre (2).

17. Dispositif selon une ou plusieurs des revendications précédentes, **caractérisé en ce que** l'élément filtrant (22) avec son avant de filtre (13) est disposé et fixé de manière à bourgeonner avec un écartement hors du manchon du filtre (2) en débordant dans le manchon du filtre (2).

18. Dispositif selon une ou plusieurs des revendications précédentes, **caractérisé en ce que** l'élément filtrant (22) comporte au moins un colorant de diagnostic lié aux fibres se dissolvant lors de l'absorption de liquide, notamment du colorant vital.

19. Dispositif selon une ou plusieurs des revendications précédentes, **caractérisé en ce que** l'élément filtrant (22) est couvert d'anticorps spécifiques pour les tumeurs.
